# EUROPÄISCHE PATENTANMELDUNG

(11) **EP 0 863 176 A1**
(43) Veröffentlichungstag der Anmeldung: **09.09.1998**
(21) Anmeldenummer: 98103926.6
(22) Anmeldetag: 05.03.1998
(51) Int. Cl.: C08J 9/20, A61K 9/50, C08F 291/00

(54) **Polymerisate mit mehr als einem Hohlraum**

(30) Priorität: 07.03.1997 DE 19709490
(71) Anmelder: BASF AKTIENGESELLSCHAFT, 67056 Ludwigshafen (DE)
(72) Erfinder: Schlarb, Bernhard, Dr., 67067 Ludwigshafen (DE); Schwarzenbach, Elmar, Dr., 67354 Römerberg (DE); Heckmann, Walter, Dr., 69469 Weinheim (DE)
(74) Vertreter: Isenbruck, Günter, Dr.

(57) **Zusammenfassung**

Polymerisat in Form von Teilchen einer Größe von mehr als 1 µm, die mehr als einen Hohlraum aufweisen, eine wäßrige Dispersion, die dieses Polymerisat enthält, Verfahren zu deren Herstellung sowie deren Verwendung.

## Beschreibung

Die vorliegende Erfindung betrifft ein Polymerisat in Form von Teilchen einer Größe von mehr als 1 µm, die mehr als einen Hohlraum aufweisen, eine wäßrige Dispersion, die dieses Polymerisat enthält, Verfahren zu deren Herstellung sowie deren Verwendung.

Polymerisate, meist in Form von wäßrigen Dispersionen, die Hohlräume aufweisen, sind an sich bekannt und werden seit einigen Jahren u. a. als deckende Weißpigmente anstelle von Titandioxid eingesetzt.

Teilchen mit nur einem Hohlraum im Kern sind u. a. in den US-Patenten 4 427 836, 4 469 852 und 4 594 363 beschrieben.

Polymerteilchen mit mehreren Hohlräumen sind ebenfalls bekannt.

So beschreibt die JP 08 89 789 die Herstellung von hohlen Harzteilchen als Ersatzstoff für TiO₂ durch Vermischen eines selbstdispergierbaren Harzes, einer nicht-mischbaren hydrophohen Substanz und einem organischen Lösungsmittel, Emulgierung dieses Gemisch, so daß sich die hydrophohe Substanz innerhalb der gebildeten Harzteilchen befindet und anschließende Extraktion der hydrophoben Substanz unter Verwendung eines geeigneten Lösungsmittels. Mit dieser Methode werden Teilchen mit einem Durchmesser von 0,5 µm erhalten.

US 4 968 562 beschreibt säurefreie Polymerteilchen mit mehr als einem Hohlraum, die (a) mindestens ungefähr 70 Gewichtsteile mindestens eines Alkylacrylats oder -methacrylats, sowie (b) bis zu 30 Gewichtsteile mindestens eines Stickstoff-enthaltenden polaren Monomers umfassen. Die dort in den Beispielen beschriebenen Polymerteilchen weisen Hohlräume mit einem Durchmesser von ungefähr 2 µm auf und werden bei der Herstellung vernetzt.

In der EP-A 0 408 189 werden Teilchen eines hydrophoben Polymers beschrieben, die mindestens einen Hohlraum aufweisen und eine im wesentlichen hydrophobe oberflächenaktive Komponente und eine im wesentlichen hydrophile oberflächenaktive Komponente umlassen. Als hydrophobe oberflächenaktive Komponente wird dort ein darin näher definiertes Blockcopolymer mit einem hydrophilen Anteil von 5 bis 45 Gew.-% eingesetzt, während die dort beschriebene hydrophile oberflächenaktive Komponente u. a. dadurch gekennzeichnet ist, daß der hydrophile Anteil innerhalb dieser Komponente 40 bis 90 Gew.-% beträgt.

Okubo et al. beschreiben ein weiteres Verfahren zur Herstellung von Latexteilchen mit vielen Hohlräumen, wobei Styrol-Butylacrylat-Methacrylsäure-Terpolymere zunächst mit Basen und dann mit Säuren behandelt werden. (s. Colloid Polym. Sci., Bd. 269, S. 1257 - 1262 (1991)). Das Verfahren gemäß dieser Druckschrift ist dahingehend nachteilig, daß große Mengen von Tensiden zur Stabilisierung der Dispersion verwendet werden müssen. Darüberhinaus werden gemäß dieser Druckschrift Polymerteilchen einer Größe von deutlich weniger als 1 µm erhalten.

Ein weiteres Verfahren zur Herstellung von Latexteilchen mit vielen Hohlräumen, das auf einem Extraktionsprozeß basiert, wird ebenfalls von Okubo et al. in Colloid Polym. Sci., Bd. 272, S. 530 - 535 (1994) beschrieben.

Hierbei werden unvernetzte Polystyrolteilchen vorgelegt und anschließend wird ein Gemisch aus Styrol und Divinylbenzol aufpolymerisiert. Durch Extraktion des unvernetzten Polystyrols mit Toluol lassen sich monodisperse, vernetzte Teilchen mit vielen Hohlräumen herstellen. Als Stabilisator wird dort Polyacrylsäure als hydrophile Komponente eingesetzt. Ferner sind die Teilchen vernetzt. Ein besonderer Nachteil des dort beschriebenen Verfahrens ist in der aufwendigen Extraktion zu sehen, die einige Tage bis zu 2 Wochen dauern kann.

Die Anmelderin selbst beschreibt in der EP-A 0 225 612 wäßrige Polymerisatdispersionen, deren Polymerisatanteil dem hier beanspruchten Polymerisat bzgl. der Zusammensetzung ähnlich ist. Gemäß den Beispielen dieser Druckschrift wird die Polymerisation ebenfalls in einem organischen, jedoch wasser-mischbaren Lösungsmittel, wie z. B. Ethanol oder Isobutanol, durchgeführt. Dabei werden Teilchen mit einer Kern-Schale-Struktur erhalten, die im allgemeinen einen Durchmesser von weniger als 1 µm besitzen.

Wie sich aus obigem ergibt, existieren bereits einige Verfahren zur Herstellung von Polymerisaten in Form von Teilchen, die mehr als einen Hohlraum aufweisen, sowie derartige Teilchen an sich.

Diese Polymerisate sind jedoch nicht in all ihren Eigenschaften zufriedenstellend und weisen zum Teil nur geringe Stabilität, unzureichende Anzahl oder zu kleine Hohlräume zur optimalen Verwendung als Pigment auf. Die Verfahren sind zum Teil dahingehend nachteilig, daß sie nur mit hohem technischen und Zeit- Aufwand ausführbar sind, oder nennenswerte Mengen an organischen Lösungsmitteln benötigen.

In Anbetracht des obigen Stands der Technik liegt der vorliegenden Erfindung die Aufgabe zugrunde, ein Polymerisat bereitzustellen, das mehr als einen Hohlraum aufweist, insbesondere für die Anwendung als Weißpigment hervorragend geeignet ist und in Form einer wäßrigen Dispersion, die frei von organischen Stabilisatoren ist, in stabiler Form existieren kann. Ferner sollte sich das hier in Rede stehende Polymerisat weitgehend frei von organischen Lösungsmitteln herstellen lassen.

Demgemäß betrifft die vorliegende Erfindung ein Polymerisat in Form von Teilchen einer Größe von mehr als 1 µm, die mehr als einen Hohlraum aufweisen, das im wesentlichen aus den Komponenten
A) 25 bis 70 Gew.-% eines Copolymerisats A enthaltend
   I) 5 bis 50 Gew.-% mindestens eines copolymerisierbaren, mindestens eine hydrophile Gruppen enthaltenden Monomers
   II) 20 bis 95 Gew.-% mindestens eines copolymerisierbaren Monomers, das frei von hydrophilen Gruppen ist,
   III) 0 bis 30 Gew.-% mindestens einer sonstigen copolymerisierbaren Verbindung
   wobei die Summe der unter (I) bis (III) genannten Prozentzahlen 100 ist,
B) 30 bis 75 Gew.-% eines Lösungspolymerisats B, das 0 bis weniger als 5 Gew.-% des obigen Monomers I enthält,
besteht,
wobei das Polymerisat erhältlich ist durch ein Verfahren, das die folgenden Stufen umfaßt:
α) Lösungspolymerisation einer der Komponenten (A) oder (B) in einem organischen Lösungsmittel und anschließende Lösungspolymerisation der anderen Komponente in der so erhaltenen Polymerisationslösung, wobei die Lösungspolymerisation in einem mit Wasser nicht mischbaren Lösungsmittel oder Lösungsmittelgemisch durchgeführt wird,
β) Dispergierung der (A) und (B) enthaltenden Lösung unter Zusatz einer Base in Wasser, und
γ) destillative Entfernung des organischen Lösungsmittels auf eine Konzentration von weniger als 5 Gew.-%, bezogen auf die Menge der Dispersion.

Die Erfindung betrifft ferner eine wäßrige Dispersion, enthaltend das obige Polymerisat.

Die Erfindung betrifft ferner ein Verfahren zur Herstellung einer wäßrigen Dispersion wie oben definiert, das die folgenden Stufen umfaßt:
α) Lösungspolymerisation einer der Komponenten (A) oder (B) in einem organischen Lösungsmittel und anschließende Lösungspolymerisation der anderen Komponente in der so erhaltenen Polymerisationslösung, wobei die Lösungspolymerisation in einem mit Wasser nicht mischbaren Lösungsmittel oder Lösungsmittelgemisch durchgeführt wird,
β) Dispergierung der (A) und (B) enthaltenden Lösung unter Zusatz einer Base in Wasser, und
γ) destillative Entfernung des organischen Lösungsmittels auf eine Konzentration von weniger als 5 Gew.-%, bezogen auf die Menge der Dispersion.

Die Erfindung betrifft ferner ein Verfahren zur Herstellung eines wie oben definierten Polymerisats als Feststoff, das die folgenden Stufen umfaßt:
α) Lösungspolymerisation einer der Komponenten (A) oder (B) in einem organischen Lösungsmittel und anschließende Lösungspolymerisation der anderen Komponente in der so erhaltenen Polymerisationslösung, wobei die Lösungspolymerisation in einem mit Wasser nicht mischbaren Lösungsmittel oder Lösungsmittelgemisch durchgeführt wird,
β) Dispergierung der (A) und (B) enthaltenden Lösung unter Zusatz einer Base in Wasser,
γ) destillative Entfernung des organischen Lösungsmittels auf eine Konzentration von weniger als 5 Gew.-%, bezogen auf die Menge der Dispersion, und
δ) anschließende Trocknung der Dispersion.

Bei dem erfindungsgemäßen Polymerisat handelt es sich um eine Polymermischung aus einem Copolymer, das reich an hydrophilen Gruppen ist, und einem Lösungspolymerisat, das arm an hydrophilen Gruppen ist, bzw. überhaupt keine hydrophilen Gruppen aufweist.

Das Polymerisat liegt im allgemeinen in Form von Teilchen einer Größe von mehr als 1 bis ungefähr 50, vorzugsweise mehr als 1 bis ungefähr 10 und insbesondere ungefähr 3 bis ungefähr 6 µm vor.

Die Hohlraumgröße ist an sich frei wählbar, solange diese es ermöglicht, daß pro Teilchen mehr als ein Hohlraum vorliegt. Vorzugsweise liegt der Durchmesser der vorhandenen Hohlräume jedoch im Bereich von ungefähr 400 bis ungefähr 800 nm, also im wesentlichen im Bereich der Wellenlänge des sichtbaren Lichts.

Vorzugsweise besitzt die erhaltene Polymermischung eine Glastemperatur, die oberhalb Raumtemperatur, weiter bevorzugt zwischen ungefähr 40 °C und ungefähr 150 °C, und insbesondere zwischen ungefähr 60 °C und 110 °C liegt, da es dann möglich ist, eine vorher gebildete Struktur bei Raumtemperatur einzufrieren.

Das erfindungsgemäße Polymerisat besteht im wesentlichen aus ungefähr 25 bis ungefähr 70, vorzugsweise ungefähr 40 bis ungefähr 60 und insbesondere ungefähr 45 bis ungefähr 55 Gew.-% eines Copolymerisats A und zu ungefähr 30 bis ungefähr 75, vorzugsweise ungefähr 40 bis 60 und insbesondere ungefähr 45 bis ungefähr 55 Gew.-% eines Lösungspolymerisats B, wobei die Summe der unter (A) und (B) genannten Prozentzahlen 100 beträgt.

Komponente (A) ist ein Copolymerisat enthaltend
I) ungefähr 5 bis ungefähr 50, vorzugsweise ungefähr 10 bis ungefähr 30 und insbesondere ungefähr 10 bis ungefähr 20 Gew.-% mindestens eines copolymerisierbaren, hydrophile Gruppen enthaltenden Monomers,
II) ungefähr 20 bis ungefähr 95, vorzugsweise ungefähr 50 bis ungefähr 90 Gew.-% eines copolymerisierbaren Monomers, das frei von hydrophilen Gruppen ist, und
III) 0 bis ungefähr 30, vorzugsweise 0 bis ungefähr 20 Gew.-% mindestens einer sonstigen copolymerisierbaren Verbindung,
wobei die Summe der unter (I) bis (III) genannten Prozentzahlen 100 ist.

Komponente (B) ist ein im wesentlichen von hydrophilen Gruppen freies Lösungspolymerisat und weist 0 bis ungefähr 5 Gew.-%, vorzugsweise 0 bis ungefähr 3 Gew.-% und insbesondere kein copolymerisierbare hydrophile Gruppen enthaltendes Monomer (I) auf. Demgemäß besteht die Komponente (B) insbesondere lediglich aus den Monomeren (II) und/oder (III).

Zur Herstellung des oben definierten Polymers wird zunächst eine der beiden Komponenten (A) und (B) in einem organischen Lösungsmittel polymerisiert, und in der so erhaltenen Polymerlösung wird dann die andere Komponente (A) oder (B) polymerisiert. Ferner ist es möglich, eine der Komponenten (A) oder (B) in einem organischen Lösungsmittel zu polymerisieren und die andere Komponente als separat hergestelltes Polymerisat in die erhaltene Polymerlösung einzubringen.

Die Komponente (B) ist, für sich allein betrachtet, nicht wasserverdünnbar und enthält, wenn überhaupt, lediglich geringe Mengen eines hydrophile Gruppen enthaltenden Monomers, wie oben definiert.

Zu den Monomeren der Komponenten (A) und (B) ist folgendes auszuführen:

Als Monomer (I) sind insbesondere copolymerisierbare olefinisch ungesättigte, bis zu 10 Kohlenstoffatome enthaltende Carbonsäuren oder Carbonsäureanhydride, wie z. B. Acrylsäure, Methacrylsäure, Maleinsäure, Itaconsäure bzw. die Anhydride oder die Halbester dieser Dicarbonsäuren zu nennen. Die Anhydridgruppen der Copolymerisate können vor der Neutralisation beispielsweise durch Erwärmen mit 1 bis 8 Kohlenstoffatomen enthaltenden Alkoholen oder Glykolethern oder Aminen in die entsprechenden Halbester- bzw. Halbamidgruppen überführt werden. Beispiele für solche Alkohole bzw. Glykolether sind Ethanol, Isopropanol, Butanol und Butylglykol. Beispiele für Amine sind NH₃, primäre Amine, wie z. B. Butylamin, und sekundäre Amine, wie z. B. Diethylamin. Selbstverständlich können auch Gemische aus zwei oder mehr der obigen Monomere eingesetzt werden.

Ferner können copolymerisierbare olefinisch ungesättigte Sulfonsäuren, wie z. B. 3-Sulfonpropyl(meth)acrylsäure oder 2-Acrylamido-2-methylpropansulfonsäure, oder einem Derivat davon copolymerisierbare olefinisch ungesättigte Sulfate, copolymerisierbare olefinisch ungesättigte Phosphorsäuren oder einem Derivat davon, wie z. B. der Phosphonsäureester von Hydroxyethyl(meth)acrylat, oder copolymerisierbare olefinisch ungesättigte Phosphonsäuren oder einem Derivat davon, wie z. B. Vinylphosphorsäure oder deren Gemische, verwendet werden.

Als besonders bevorzugte Monomere (I) sind Acrylsäure, Methacrylsäure und Itaconsäure zu nennen.

Als Monomer (II) sind zu nennen:
Ester der Acrylsäure oder Methacrylsäure mit 1 bis 20 Kohlenstoffatome enthaltenden geradkettigen oder verzweigtkettigen Monoalkanolen, wie z. B. Methylacrylat, Ethylacrylat, Isopropylacrylat, Methylmethacrylat, n-Butylacrylat, n-Butylmethacrylat, Isobutylacrylat, Isobutylmethacrylat, tert.-Butylacrylat, 2-Ethylhexylacrylat, 2-Ethylhexylmethacrylat, Laurylacrylat, sowie deren Gemische, wobei n-Butylacrylat und Methylmethacrylat besonders bevorzugt sind;
vinylaromatische Verbindungen, die gegebenenfalls insbesondere am aromatischen Ring ein- oder mehrfach durch mindestens einen Alkylrest und/oder Halogen und/oder mindestens ein Aminorest substituiert sein können, wie z. B. Vinyltoluol, Styrol, α- und p-Methylstyrol, α-Butylstyrol, 4-n-Butylstyrol, 4-n-Decylstyrol, wobei Styrol bevorzugt ist.

Es können auch Gemische aus zwei oder mehr davon verwendet werden.

Bei dem Monomer (III) handelt es sich um weitere, unter (I) und (II) nicht genannte copolymerisierbare olefinisch ungesättigte Verbindungen.

Zu nennen sind dabei:
Vinylester von Carbonsäuren mit 1 bis 20 C-Atomen, wie z. B. Vinyllaurat, Vinylstearat, Vinylpropionat und Vinylacetat;
Mono(meth)acrylate von Alkandiolen, wie z. B. Hydroxyethyl- und Hydroxypropyl(meth)acrylat sowie Butandiolmono(meth)acrylat;
Amide wie z. B. (Meth)acrylamid;
Vinylether, Vinylester, Diester der Maleinsäure, Itaconsäure, Citraconsäure oder Mesaconsäure mit, ggf. Ether- oder Thioether-Gruppen enthaltenden Monoalkoholen mit 1 bis 20 Kohlenstoffatomen;
Nitrile, wie z. B. Acrylnitril und Methacrylnitril;
Vinylhalogenide, d. h. mit Chlor, Fluor oder Brom substituierte ethylenisch ungesättigte Verbindungen, wie z. B. Vinylchlorid oder Vinylidenchlorid;
nicht-aromatische Kohlenwasserstoffe mit 2 bis 8 C-Atomen und ein oder zwei olefinischen Doppelbindungen, wie z. B. Butadien, Isopren, Chloropren, Ethylen, Propen, Buten, Penten, Hexen, Isobuten;
Diolefine, wie z. B. Butadien und Isopren;
   sowie Gemische aus zwei oder mehr davon.

Wie sich aus obigem ergibt, wird im Rahmen der vorliegenden Erfindung vorzugsweise ohne Emulgator gearbeitet, wobei jedoch auch die Verwendung handelsüblicher Emulgatoren nicht ausgeschlossen ist.

Bezüglich der im Rahmen des erfindungsgemäßen Verfahrens verwendbaren Lösungsmittel existieren keinerlei Beschränkungen, solange diese bei der Polymerisationstemparatur mit Wasser nicht mischbar sind, d. h. mit diesem Medium eine Mischungslücke aufweisen.

Zu nennen sind hierbei insbesondere aromatische Kohlenwasserstoffe, wie z. B. Toluol oder Xylol; aliphatische Kohlenwasserstoffe, wie z. B. n-Hexan, n-Heptan, Iso- und n-Octan; cycloaliphatische Kohlenwasserstoffe, wie z. B. Cyclohexan; oder Gemische aus zwei oder mehr davon. Besonderes gute Resultate werden erhalten, wenn es sich bei dem verwendeten organischen Lösungsmittel um ein Gemisch zwischen einem für das hier in Rede stehende Polymerisat guten Lösungsmittel, wie z. B. Toluol, und einem für das Polymerisat sehr schlechten Lösungsmittel (Fällungsmittel), wie z. B. n-Octan, handelt. Insbesondere das Gemisch aus Toluol und n-Octan hat sich als besonders vorteilhaft einsetzbar erwiesen, wobei mit einem Verhältnis Toluol zu n-Octan von ungefähr 5:1 bis ungefähr 1:1 die besten Resultate erzielt werden.

Die Polymerisation erfolgt zweckmäßigerweise in Gegenwart von ungefähr 0,3 bis ungefähr 5,0, vorzugsweise ungefähr 0,5 bis ungefähr 3,0 Gew.-%, bezogen auf die Summe der Monomeren (I) bis (III), radikalbildender Initiatoren, wie z. B. Azobiscarbonsäureamiden, Azobiscarbonsäurenitrilen oder Peroxiden, im allgemeinen bei Temperaturen zwischen ungefähr 50 und ungefähr 150 °C, vorzugsweise zwischen ungefähr 80 und ungefähr 130 °C, gegebenenfalls in Gegenwart von Reglern, wie z. B. Mercaptoethanol, Tertiärdodecylmercaptan oder Diisopropylxanthogendisulfid, die in Mengen von 0 bis ungefähr 3 Gew.-%, bezogen auf die Summe der Monomeren (I) bis (III) vorhanden sein können.

Es ist dabei nicht von Bedeutung, ob zuerst die Komponente (A) und dann die Komponente (B) polymerisiert wird oder ob umgekehrt vorgegangen wird. In einer bevorzugten Ausführungsform der Erfindung wird jedoch zunächst die Komponente (A) und anschließend die Komponente (B) polymerisiert. Die zuerst hergestellte Komponente wird dabei weitgehend, d. h. vorzugsweise zu mehr als ungefähr 80 %, insbesondere zu mehr als ungefähr 90 %, auspolymerisiert, bevor mit der Polymerisation der zweiten Komponente begonnen wird.

Nach dem Ende der Polymerisation wird die (A) und (B) enthaltende Lösung unter Zusatz einer Base, wie z. B. Ammoniak, in Wasser dispergiert und dabei strukturviskos oder plastisch fließend eingestellt. Dabei entsteht zunächst, da die organische Phase mit Wasser nicht mischbar ist, eine Wasser-in-Öl(WO)-Emulsion, d. h. in der organischen Phase sind kleine Wassertröpfchen emulgiert. Bei weiterer Wasser- bzw. Basenzugabe wird bei einem bestimmten Phasenverhältnis (Wasserphase/organische Phase) der Phaseninversionspunkt erreicht, an dem die WO-Emulsion in eine WOW-Emulsion "umkippt". Es sind dann Öltröpfchen (organische Phase) in der Wasserphase emulgiert, die selbst wiederum kleine Volumina aus Wasser eingeschlossen enthalten. Der Phaseninversionspunkt kann durch Leitfähigkeitsmessungen, die dem Fachmann bekannt sind, bestimmt werden.

Dabei wird die Bildung von Polymerisaten, die mehr als einen Hohlraum aufweisen, bzw. die Bildung einer WOW-Emulsion durch eine hohe Viskosität der organischen Phase von vorzugsweise ungefähr 10 bis ungefähr 1000, weiter bevorzugt ungefähr 50 bis ungefähr 500 Pa.s, jeweils bei einer Scherrate von 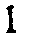 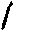 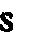 (s= Sekunde) und einer Temperatur von 80 °C und durch Abkühlen während des Dispergierens begünstigt, so daß eine Reaktionsführung unter Verwendung einer organischen Phase mit der oben definierten Viskosität bei gleichzeitigem Abkühlen während des Dispergierens bevorzugt ist.

Die resultierende Mischung der Komponenten (A) und (B) wird nach Neutralisation mit einer Base, vorzugsweise Ammoniak, durch Zugabe von Wasser in eine Dispersion überführt.

Anschließend wird das organische Lösungsmittel oder das organische Lösungsmittelgemisch destillativ entfernt, wobei der Gehalt des organischen Lösungsmittels in der schlußendlich erhaltenen Dispersion in einer Konzentration von vorzugsweise weniger als 5 Gew.-%, insbesondere weniger als 3 Gew.-%, bezogen auf die Menge der Dispersion beträgt.

Der Neutralisationsgrad der so erhaltenen Dispersion beträgt im allgemeinen ungefähr 10 bis ungefähr 150 %, vorzugsweise ungefähr 15 bis ungefähr 100 %, besonders bevorzugt ungefähr 40 bis ungefähr 70 %. Der pH-Wert der Dispersion kann demnach zwischen ungefähr 6 und ungefähr 10, vorzugsweise zwischen ungefähr 7,0 und 9,0 betragen.

Der Polymerisatgehalt der erfindungsgemäßen Dispersion wird zweckmäßigerweise so gewählt, daß sich eine für den Verarbeiter günstige Viskosität ergibt. Der Polymerisatgehalt beträgt deshalb im allgemeinen ungefähr 25 bis ungefähr 60 Gew.-%, vorzugsweise ungefähr 30 bis 40 Gew.-%.

Um das Polymerisat als Feststoff zu erhalten, wird aus der nach dem Entfernen des organischen Lösungsmittels erhaltenen Dispersion das Wasser, z. B. durch Sprühtrocknung entfernt, d. h. die Dispersion wird getrocknet.

Das so erhaltene Polymerisat in trockener Form kann dann gelagert und als Feststoff vertrieben und verarbeitet werden.

Darüber hinaus können das erfindungsgemäße Polymerisat, die erfindungsgemäße Dispersion, bzw. das erfindungsgemäß hergestellte Polymerisat oder die erfindungsgemäß hergestellte Dispersion, wie oben definiert, als Pigment und zur Mikroverkapselung verwendet werden.

Bei der Verwendung als Pigment wird das erfindungsgemäße Polymerisat bzw. die erfindungsgemäße Dispersion mit weiteren Komponenten, z.B. einer Anstrichfarbe, wie z.B. Bindemitteln und sonstigen Lack-Hilfsstoffen, vermischt und somit eine das erfindungsgemäße Polymerisat als Pigment umfassende Zusammensetzung erhalten.

Bei der Mikroverkapselung wird das erfindungsgemäße Polymerisat bzw. die erfindungsgemäße Dispersion mit einem geeigneten Substrat, wie z.B. einem Pigment, einer z.B. pharmakologisch oder agrochemisch wirksamen Verbindung, in Kontakt gebracht, wobei das Substrat sich dann in den Hohlräumen des erfindungsgemäßen Polymerisats einlagert.

Demgemäß betrifft die vorliegende Erfindung auch ein Verfahren zur Mikroverkapselung von Substraten, das dadurch gekennzeichnet ist, daß ein Substrat mit dem erfindungsgemäßen Polymerisat bzw. der erfindungsgemäßen Dispersion in Kontakt gebracht wird.

Demgemäß betrifft die vorliegende Erfindung auch ein Verfahren zur Herstellung einer Zusammensetzung, die das erfindungsgemäße Polymerisat als Pigment enthält, wobei das erfindungsgemäße Polymerisat oder die erfindungsgemäße Dispersion mit weiteren für die Herstellung von Pigmententhaltenden Zusammensetzungen verwendbaren Komponenten, wie oben definiert, vermischt wird.

Darüber hinaus betrifft die vorliegende Erfindung die oben beschriebenen Zusammensetzungen an sich.

### Beispiel

Zunächst wurden 119 g Toluol und 51 g n-Octan in einem 4-Liter-Reaktionskolben, der mit einem Ankerrührer, Rückflußkühler und zwei Zuläufen ausgestattet war, vorgelegt und zusammen mit 182 g von Zulauf 1, der aus 75 g Acrylsäure und 425 g Styrol bestand, unter Stickstoff auf eine Temperatur von 105 °C aufgeheizt. Nach Erreichen dieser Temperatur wurden 15 g eines Zulaufs 3, der aus 20 g tert.-Butylperoctoat, 161 g Toluol und 69 g n-Octan bestand, in 2 Minuten zudosiert. Anschließend wurde die verbliebene Menge von Zulauf 1 (318 g) und 15 g von Zulauf 3 in 45 Minuten zudosiert. Es wurde noch 15 Minuten nachpolymerisiert. Dann wurden 135 g von Zulauf 2 (500 g Styrol) und 30 g von Zulauf 3 in zwei Stunden zudosiert. Anschließend wurde eine Stunde nachpolymerisiert. Nun wurde der Rest von Zulauf 2 (365 g) und 130 g von Zulauf 3 in drei Stunden zudosiert. Anschließend wurde der Rest von Zulauf 3 in zwei Stunden zudosiert und noch zwei Stunden lang nachpolymerisiert.

Die Polymerlösung wurde bei einer Außentemperatur von 70 °C mit 70,8 g wäßriger Ammoniaklösung (25 Gew.-%ig) neutralisiert. Anschließend wurde durch Einrühren von 1000 g Wasser innerhalb von einer Stunde dispergiert.

Bei einer Außentemperatur von 70 °C wurden im Vakuum 835 g eines Lösungsmittel/Wasser-Gemischs abdestilliert und gleichzeitig 1200 g frisches Wasser zudosiert.

Es wurde eine Dispersion mit folgenden Eigenschaften erhalten:
pH-Wert: 8,1
Feststoffgehalt: 32,9 %,
Restgehalt an Lösungsmittel: 0,3 %,
Teilchengröße: > 1 bis ungefähr 5 µm (elektronenmikroskopische Bestimmung)

## Patentansprüche

1. Polymerisat in Form von Teilchen einer Größe von mehr als 1 µm, die mehr als einen Hohlraum aufweisen, das im wesentlichen aus den Komponenten
A) 25 bis 70 Gew.-% eines Copolymerisats A enthaltend
I) 5 bis 50 Gew.-% mindestens eines copolymerisierbaren, mindestens eine hydrophile Gruppe enthaltenden Monomers
II) 20 bis 95 Gew.-% mindestens eines copolymerisierbaren Monomers, das frei von hydrophilen Gruppen ist,
III) 0 bis 30 Gew.-% mindestens einer sonstigen copolymerisierbaren Verbindung
wobei die Summe der unter (I) bis (III) genannten Prozentzahlen 100 ist,
B) 30 bis 75 Gew.-% eines Lösungspolymerisats B, das 0 bis weniger als 5 Gew.-% des obigen Monomers I enthält,
besteht,
wobei das Polymerisat erhältlich ist durch ein Verfahren, das die folgenden Stufen umfaßt:
α) Lösungspolymerisation einer der Komponenten (A) oder (B) in einem organischen Lösungsmittel und anschließende Lösungspolymerisation der anderen Komponente in der so erhaltenen Polymerisationslösung, wobei die Lösungspolymerisation in einem mit Wasser nicht mischbaren Lösungsmittel oder Lösungsmittelgemisch durchgeführt wird,
β) Dispergierung der (A) und (B) enthaltenden Lösung unter Zusatz einer Base in Wasser, und
γ) destillative Entfernung des organischen Lösungsmittels auf eine Konzentration von weniger als 5 Gew.-%, bezogen auf die Menge der Dispersion.

2. Polymerisat nach Anspruch 1, wobei das Monomer I ausgewählt wird unter einer copolymerisierbaren olefinisch ungesättigten Carbonsäure oder deren Anhydrid, einer copolymerisierbaren olefinisch ungesättigten Sulfonsäure oder einem Derivat davon, einem copolymerisierbaren olefinisch ungesättigten Sulfat, einer copolymerisierbaren olefinisch ungesättigten Phosphorsäure oder einem Derivat davon, einer copolymerisierbaren olefinisch ungesättigten Phosphonsäure oder einem Derivat davon, jeweils mit bis zu 10 C-Atomen, und einem Gemisch aus zwei oder mehr davon.

3. Polymerisat nach Anspruch 1 oder 2, wobei das Monomer II ausgewählt wird unter einem (Meth)acrylsäurealkylester mit einem C₁ bis C₂₀-Alkylrest, einer vinylaromatischen Verbindung, und einem Gemisch aus zwei oder mehr davon.

4. Polymerisat nach einem der Ansprüche 1 bis 3, wobei die Größe der Hohlräume im Bereich von 400 bis 800 nm liegt.

5. Wäßrige Dispersion, enthaltend mindestens ein Polymerisat nach einem der Ansprüche 1 bis 4.

6. Verfahren zur Herstellung einer wäßrigen Dispersion wie in Anspruch 5 definiert, das die folgenden Stufen umfaßt:
α) Lösungspolymerisation einer der Komponenten (A) oder (B) in einem organischen Lösungsmittel und anschließende Lösungspolymerisation der anderen Komponente in der so erhaltenen Polymerisationslösung, wobei die Lösungspolymerisation in einem mit Wasser nicht mischbaren Lösungsmittel oder Lösungsmittelgemisch durchgeführt wird,
β) Dispergierung der (A) und (B) enthaltenden Lösung unter Zusatz einer Base in Wasser, und
γ) destillative Entfernung des organischen Lösungsmittels auf eine Konzentration von weniger als 5 Gew.-%, bezogen auf die Menge der Dispersion.

7. Verfahren zur Herstellung eines Polymerisats wie in einem der Ansprüche 1 bis 4 definiert als Feststoff, das die folgenden Stufen umfaßt:
α) Lösungspolymerisation einer der Komponenten (A) oder (B) in einem organischen Lösungsmittel und anschließende Lösungspolymerisation der anderen Komponente in der so erhaltenen Polymerisationslösung, wobei die Lösungspolymerisation in einem mit Wasser nicht mischbaren Lösungsmittel oder Lösungsmittelgemisch durchgeführt wird,
β) Dispergierung der (A) und (B) enthaltenden Lösung unter Zusatz einer Base in Wasser,
γ) destillative Entfernung des organischen Lösungsmittels auf eine Konzentration von weniger als 5 Gew.-%, bezogen auf die Menge der Dispersion, und
δ) anschließende Trocknung der Dispersion.

8. Verwendung eines Polymerisats, wie in einem der Ansprüche 1 bis 4 definiert, oder eines Polymerisats hergestellt mittels des Verfahrens gemäß Anspruch 7 oder einer Dispersion gemäß Anspruch 5 oder einer Dispersion hergestellt mittels des Verfahrens gemäß Anspruch 6, als Pigment und zur Mikroverkapselung.
